# EUROPEAN PATENT SPECIFICATION

(11) **EP 3 195 852 B1**
(45) Date of publication and mention of the grant of the patent: **27.01.2021**
(21) Application number: 16835338.1
(22) Date of filing: 25.07.2016
(51) Int. Cl.: A61K 8/02, A61K 8/365, A61K 8/92, A61Q 19/00, A61K 8/42, A61K 8/34

(54) **KERATIN SOFTENING PATCH**
PFLASTER ZUM WEICHMACHEN VON KERATIN
TIMBRE ADOUCISSANT À LA KÉRATINE

(30) Priority: 13.08.2015 KR 20150114283
(43) Date of publication of application: 26.07.2017
(73) Proprietor: Wooshin Labottach Co., Ltd., Guro-gu Seoul 08390 (KR)
(72) Inventor: NAM, Tack Soo, Seoul 08390 (KR)
(74) Representative: Louis Pöhlau Lohrentz
(86) International application number: PCT/KR2016/008088
(87) International publication number: WO 2017/026698

(56) References cited:
- WO-A2-2011/050947
- JP-A- 2004 123 662
- JP-A- 2012 012 374
- KR-A- 20100 061 881
- KR-A- 20110 013 000
- KR-A- 20110 051 392
- KR-A- 20110 082 992
- KR-A- 20130 138 550
- KR-U- 20090 003 762
- DATABASE GNPD [Online] MINTEL; 1 May 2015 (2015-05-01), Watsons Beauty Buffet: "Moisturizing Foot Mask", XP002781202, Database accession no. 3189111
- DATABASE GNPD [Online] MINTEL; 1 October 2011 (2011-10-01), Kobayashi Healthcare: "Overnight Cracked Heel Care", XP002781203, Database accession no. 1653136
- DATABASE GNPD [Online] MINTEL; 1 March 2015 (2015-03-01), Dr. Douxi Biochemical Technology: "Lactic Acid Foot Cloth", XP002781204, Database accession no. 3079077
- DATABASE GNPD [Online] MINTEL; 1 December 2011 (2011-12-01), Chemcorp: "Exfoliating Foot Peel", XP002781205, Database accession no. 1670888

## Description

### TECHNICAL FIELD

The present invention relates to a patch for softening dead skin cells.

### BACKGROUND ART

### [Dead skin cells]

The human skin (integument) is composed of three layers of tissue: the outer layer of epidermis made up of keratinocytes, the middle layer of dermis containing collagen fiber and elastic fiber, and the inner fat (subcutaneous tissue) layer comprised of fat cells. The outermost thin layer of the skin is referred to as epidermis, providing the initial barrier to the external environment. According to the stage of the keratinocyte differentiation process, the epidermis includes, from deepest to most superficial, basal layer, spinous layer, granular layer, clear/translucent layer, and cornified layer (*stratum corneum*).

Keratinocytes, constituting the epidermal layer of the skin, begin their life cycle in the basal layer and move towards the surface, through the spinous layer, granular layer, etc., changing in structure from one layer of the skin to the next as differentiation progresses. Older keratinocytes, cornified as the result of migration towards the skin surface and differentiation, reach the outermost layer of the epidermis, the *stratum corneum,* and then slough off the skin. This process is called "keratinization".

The keratinization process normally occurs in the outer layers of the skin all the time and repeats every 4 weeks in a normal adult, even though the period of its cycle varies from person to person. When the dead skin cells are not sloughed off due to some reasons, such as a lack of moisture and accumulate on the surface of the epidermis, they become harder to make the skin rough. Further, such an excess of the dead skin cells prevents the entry of moisture into the skin and inhibits the natural exfoliation of dead skin cells.

For prevention of this problem, there are some products commercially available to remove dead skin cells and keep the skin soft.

### [Exfoliants on the market]

General exfoliants commercially available are largely divided into two categories.

Those in the one category are chemical exfoliants, which remove dead skin cells by using alpha-hydroxy acid (AHA), beta-hydroxy acid (BHA), salicylic acid, or plant extracts containing such components to chemically dissolve dead skin cells or shortening the period of the keratinization cycle. These products make a good effect to exfoliate in a defined period of time, yet cause some side effects such as skin irritations due to their chemically active ingredients having low pH value.

Those in the other category are physical exfoliants, which are cream or gel formulas used in combination with wax or scrubs for physical exfoliation. These products cause no skin irritation in contrast to the chemical exfoliants. But, they involve the friction on the skin with wax or scrubs used to forcedly exfoliate, causing pains, and thus are not available for the sensitive skins with redness, irritation, inflammatory acne, etc. Further, they make little effect for the hardened dead skin cells such as those on the skin with seborrheic dermatitis.

### [Exfoliant-related Patent]

KR Registered Patent Publication No. 10-0570498 discloses an exfoliant product used with minimized chemical and physical stimulations on the skin. More specifically, this product is a lotion formula containing a film-forming resin like acrylate copolymer having the effects of sebum adsorption and exfoliation with a relatively low chemical stimulation on the skin in combination with a powder like polyethylene or vegetable cellulose having the exfoliation effect with a relatively low physical stimulation on the skin. The product is applied to the skin, which is then simply washed with water to remove dead skin cells.

Mintel database GNPD, 1 October 2011, Kobayashi Healthcare: "Overnight Cracked Heel Care", XP002781203, database accession no. 1653136, discloses an intensive repair patch to moisturize and repair cracked heels during sleep.

Mintel database GNPD, 1 December 2011, Chemcorp: "Exfoliating Foot Peel", XP002781205, database accession no. 1670888, discloses a softsole exfoliating foot peel to give softer skin and to remove dry, cracked skin from the feet.

### DISCLOSURE OF INVENTION

The defoliant product disclosed in KR Registered Patent Publication No. 10-0570498 has an exfoliating effect on the skin such as of the face with the cornified layer relatively not thick due to regular cleansing and control, but it displays an insignificant effect to exfoliate on the skin such as of the heels having the cornified layer relatively thick with accumulated dead skin cells.

Accordingly, the inventors of the present invention have been working on the elaboration of the plan to develop exfoliant products having an effective exfoliating action on the skin such as of the heels with the cornified layer relatively thick due to accumulated dead skin cells, yet relatively less chemical or physical irritations.

An excess of dead skin cells need to be sloughed off as they produce adverse effects on the skin in view of skin beauty and obstruct the absorption of water into the skin. An appropriate existence of dead skin cells prevents evaporation of moisture from the skin and has an initial barrier action on the skin against irritations caused by external toxic substances, so over-exfoliation can make an unintended effect to eliminate the appropriate existence of dead skin cells. Under the consideration of this fact, it is an object of the present invention to provide an exfoliant product having both exfoliating and moisturizing actions on the skin.

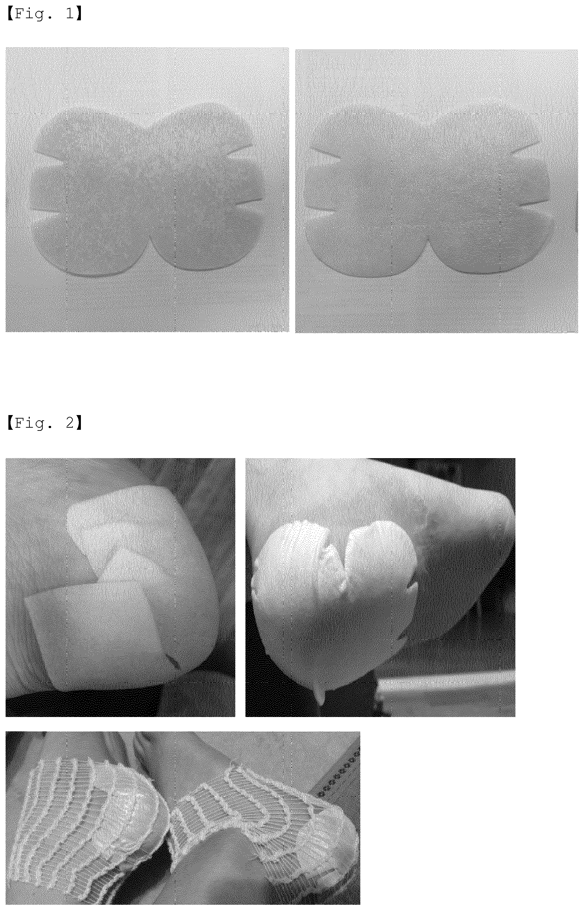

The object of the present invention may be achieved by way of the following means:
(1) There is provided a patch for exfoliation according to claim 1.
(2) The patch for exfoliation as claimed in any one of claims 1, 2 or 3 further comprises 40 to 50 wt.% of purified water and 10 to 20 wt.% of glycerin with respect to the total weight of the composition.
(3) The patch for exfoliation as claimed in any one of claims 1 to 4 comprises 1 to 5 wt.% of olive oil, 1 to 5 wt.% of jojoba oil derived from jojoba seeds, and 0.1 to 0.5 wt.% of tea tree oil derived from tea tree leaves with respect to the total weight of the composition.
(4) In the patch for exfoliation as claimed in claim 1, the support is a waterproof fiber sheet.
(5). In the patch for exfoliation as claimed in claims 1 to 7, the support has the shape of FIG. 1.
(6). There is also provided a kit for exfoliation that comprises the patch as claimed in any one of claims 1 to 7 and an elastic net fixer.

In accordance with one embodiment of the present invention, the patch preparation having a coating of cataplasma on a support offers an effective exfoliation action on the skin such as of the heels that is likely to dry due to lack of moisturization and vulnerable to keratinization to accumulate dead skin cells, yet making relatively little chemical or physical stimulations on the skin and effective moisturizing actions.
Further, the patch of the present invention contains active ingredients like urea and/ lactic acid that soften dead skin cells in combination with the moist environment, so it is available to slough off the cornified layer and also effective to maintain the skin-softening function due to the amazing synergic effect of the skin-softening ingredients and a natural moisturizing oil. In addition, a coating may be applied to the one side of a fabric sheet in accordance with the present invention. In this regard, it is possible to reduce the back-diffusion into the fabric sheet and suppress evaporation of water and volatilization of the volatile substances towards the fabric sheet, resulting in the maximized effects on the skin. It also offers good effects to moisturize the skin and maintain the skin-softening retention time.

Particularly, in accordance with one embodiment of the present invention, the support to which a coating of the cataplasma composition is applied is a waterproof fabric sheet, so it can prevent the cataplasma composition from drying out in a defined period of time while topically applied on a target skin area, not only softening dead skin cells, but also providing enough moisture for the skin to have the skin moisturized for a long time.

In addition, the embodiment of the present invention may be designed to take the shape of a butterfly as illustrated in FIG. 1, so it is easy to apply on the skin such as of the heels with 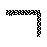 -shaped curvature and effective to offer the intended actions.

### BRIEF DESCRIPTION OF DRAWINGS

FIG. 1 shows the use of a waterproof fabric sheet (right side) as a support in comparison of a general fabric sheet (left side) to observe whether part of the cataplasma component leaks out of the support.
FIG. 2 shows the patch design of the exfoliant product according to one embodiment of the present invention (top right side) and the kit of the present invention (bottom side) applied on the skin in comparison of a conventional exfoliant product (top left side).
FIG. 3 shows the comparison in the effect between the patch of Example (right side) and that of Comparison Example (left side) before and after application on the skin.

### BEST MODES FOR CARRYING OUT THE INVENTION

The present invention is directed to an exfoliating cataplasma composition, an exfoliating patch, and an exfoliating kit.

### [Definition of terms]

As used herein, the singular forms are intended to include the plural forms as well, unless the context clearly indicates otherwise. For instance, the word "oil" as used herein means to include both a single oil component and a combination of at least two oil components, and the word "support" as used herein means to include both a single-layered support and a multilayered support.

The term "cataplasma composition" as used herein has the dictionary meanings in terms of the technological conception and refers to a composition like PAS cataplasma that contains moisture. The ingredients displaying the features of cataplasma and their combination are publicly known. Such a cataplasma composition may contain any type of skin care components such as exfoliating components unless they inhibit the features of cataplasma as specified in the General Requirements for Pharmaceutical Preparation of the Korean Pharmacopoeia (KP).

The term "natural moisturizing oil" as used herein refers to one of cosmetic oil components in contrast to the liquid type hydrocarbon-based mineral oil derived from petroleum. Generally, the natural moisturizing oil is a plant-derived component, of which the type is publicly known in the fields of cosmetic industries. The natural moisturizing oil is known to have good effects to prevent water loss from the skin, keeping the skin moist, and also to protect the skin against atopic disease or other skin problems.

The term "exfoliation", "exfoliating" or "exfoliant" as used herein inclusively means the general skin care related to dead skin cells, such as softening and removing dead skin cells and moisturizing the skin.

The term "waterproof fabric sheet" as used herein refers to a fabric sheet with waterproofing function, including a fabric sheet having an action to prevent the penetration of water. For example, a polyethylene (PE) or polyurethane (PU) film is attached to a fabric sheet made of polyethylene (PE) using heat during the preparation and an adhesive after the preparation.

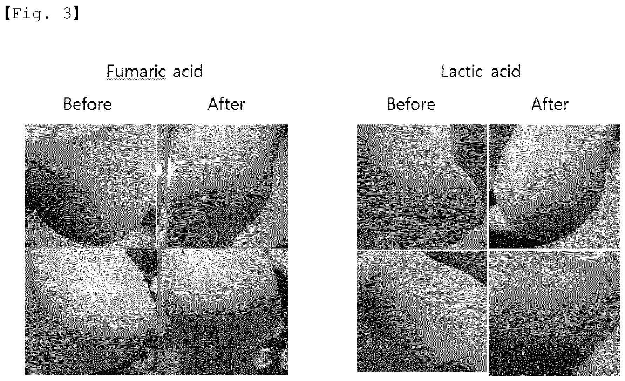

The term "elastic net fixer" as used herein refers to an auxiliary means for helping the patch of the present invention fixedly attached to a target skin area, like an elastic net as shown in the bottom side of FIG. 3.

Unless specified otherwise, the other technical and scientific terms as used herein have the same meanings as terms that are generally understood by those skilled in the art.

### [Cataplasma Composition]

Urea, naturally produced as a metabolic protein byproduct in the human body, has been put into therapeutic uses since the discovery in 1932 that it is practically non-toxic, unlikely to cause allergic reactions and effective to have important functions on the surface of the skin.

Urea has also been used as a moisturizing agent for cosmetic purposes since 1968, when it was reported that urea applied on the dry skin could display a moisturizing action on the cornified layer of the skin.

If the related mechanism is not clearly disclosed yet, urea is able to attract water and bound to it, and a report reveals that keratin has a tendency to stick to more water in the presence of urea. Of course, such a moistening agent as glycerol or propylene glycol is also likely to stick to water, but it peculiarly has the risk of attracting water from the cornified layer of the dry skin, making the skin far drier. In contrast, urea applied on the cornified layer of the normal skin or the dry skin increases the water content of the skin far more noticeably than glycerol or the like. In other words, urea has such a strong moisturizing ability irrespective of the skin type as to provide water for the cornified layer of the skin and make the skin smooth.

In recent years, urea has also been used in the exfoliating compositions since some research studies reported that urea can display an exfoliating action by loosing the space and cell membranes between keratinocytes and help the penetration of salicylic acid known as an exfoliating substance into the skin to strengthen the exfoliating action of salicylic acid.

For example, KR Laid-Open Patent Publication No. 10-2011-0082992 discloses a composition for skin exfoliation and moisturization that uses a combination of salicylic acid and urea. But the salicylic acid may cause irritations to the skin such as redness or rash, so its topic application to the skin requires caution and is preferably unacceptable.

For another example, a synergic effect of a derivative of lactic acid and urea is starting to attract some attention. KR Laid-Open Patent Publication No. 10-2011-0013000 specifies that the derivative of lactic acid for reducing dead skin cells and the urea for increasing the water content can make a synergic effect. In addition, KR Registered Patent Publication No. 10-1055963 gives attention to the exfoliating action of a combination of lactic acid or urea and an extract of the flower of prickly pear cactus (*Opuntia ficus indica var. saboten*).

Unfortunately, the example of KR Laid-Open Patent Publication No. 10-2011-0013000 or KR Registered Patent Publication No. 10-1055963 cannot display an exfoliating effect strong enough on the heels having the cornified layer relatively thickened with the accumulated dead skin cells. Such a poor exfoliating effect supposedly comes down to the fact that the epidermal penetration efficiency of the component such as urea is deteriorated on the skin such as of the heels having the cornified layer relatively thick and hard rather than on the skin having the cornified layer relatively thin.

According to a replication of experiment, the inventors of the present invention have recognized the fact that the composition containing potassium lactate, urea, carbomer, sodium hydroxide and distilled water according to Example 1 in KR Laid-Open Patent Publication No. 10-2011-0013000 entirely increases the irritations to the skin when the content of potassium lactate or urea is increased to different concentrations for the sake of improving the exfoliating effect. As a result, the above composition using a combination of potassium lactate and urea is not easy to use, because the lower content of potassium lactate and/or urea to reduce skin irritations results in a poor effect in softening the dead skin cells, whereas the higher content of potassium lactate and/or urea to make a strong action to soften the dead skin cells ends up with severe damage on the skin due to skin irritations.

In other words, there are limits as to what the conceptions disclosed in the prior patents can do in reducing skin irritations and displaying a good effect to soften the dead skin cells on the skin such as of the heels having the cornified layer relatively thick.

Changing the conception under such circumstances, the inventors of the present invention have contrived a novel cataplasma composition using a natural oil in addition to lactic acid and urea, which composition surprisingly displays a very strong exfoliating effect, maintains its exfoliating effect for a long time and makes the skin feel light, comfortable and easy with it.

Hereinafter, a description will be given as to the composition according to the preferred embodiment of the present invention.

The present invention contains cataplasma components and exfoliating components. Most of all, the exfoliating components will be described.

The patch of the present invention comprises lactic acid, urea, and a natural moisturizing oil as claimed in claim 1. The inventors of the present invention have found out the remarkable synergic effect of lactic acid and the natural moisturizing oil, ending up with the completion of the present invention.

Exceptionally as a substance used for chemical exfoliation, lactic acid that is an alpha-hydroxy acid (AHA) displays good effects in the promotion of skin moisturization, reduction of wrinkles, and treatment and/or prevention of acne, yet with no irritation to the skin, and it is widely used in the fields of cosmetic or pharmaceutical industries and commercially available on the market. In the present invention, the lactic acid not only has an exfoliating action, but serves as a catalyst in the formation of cataplasma. The lactic acid is contained in an amount of 0.5 to 1.0 wt.% with respect to the total weight of the composition. When the content of the lactic acid is less than 0.5 wt.%, it results in an insignificant skin-softening effect. When the content of the lactic acid is greater than 1.0 wt.%, it obstructs the formation of cataplasma and causes severe irritations to the skin.

Urea has been widely used in many functional cosmetic applications, because it is a substance non-toxic to the skin, highly liable to penetrate into the skin and retain water on the cornified layer of the skin and effective to change the intensified differentiation of cells to normal, offer an antibacterial effect against chronic eczema and promote the penetration of other moisturizing ingredients into the skin. The urea as used herein may include any one of known urea formulas, concentrated and isolated from urine or obtained by industrial synthesis. In the present invention, the urea not only has an exfoliating action, but serves as an acidity regulator (also called "pH control agent") in the formation of cataplasma. The urea is contained in an amount of 5 to 10 wt.% with respect to the total weight of the composition. When the content of the urea is less than 5 wt.%, it makes an insignificant skin-softening effect. When the content of the urea is greater than 10 wt.%, it obstructs the formation of cataplasma and causes severe irritations to the skin.

The natural moisturizing oil functions to promote the skin-softening effect of the lactic acid and induces natural exfoliation of dead skin cells to suppress the build-up of dead skin cells. Further, the natural moisturizing oil is effective not only to smooth down the skin under stress of the softening action, but also to moisturize the skin, so it offers an excellent function to keep the skin moist. In view of achieving the object of the present invention, it is preferable that the natural moisturizing oil as used herein includes, but is not specifically limited to, at least one selected from the group consisting of olive oil, jojoba oil derived from jojoba seeds and tea tree oil derived from tea tree leaves. The olive oil is known to have an antioxidant action and a skin-moisturizing action. Preferably, the olive oil is contained in an amount of 1 to 5 wt.% with respect to the total weight of the composition. The jojoba oil derived from jojoba seeds is known to have an outstanding effect in protecting the skin and preferably contained in an amount of 1 to 5 wt.% with respect to the total weight of the composition. The tea tree oil derived from tea tree leaves is reported to have an antioxidant effect, improve skin problems and protect the skin against atopic disease. Preferably, the tea tree oil is contained in an amount of 0.1 to 0.5 wt.% with respect to the total weight of the composition. When the content of the natural moisturizing oil is less than the lower limits, it fails to make a good synergic effect with lactic acid, but displays an insignificant effect in skin protection and moisturization. When the content of the natural moisturizing oil is greater than the upper limits, it makes the skin feel disturbingly sticky and causes severe irritations due to strong odor.

The present invention further contains purified water and glycerin in order to maximize the moisturizing effect. Preferably, the purified water is contained in an amount of 40 to 50 wt.% and the glycerin is contained in an amount of 10 to 20 wt.%, with respect to the total weight of the composition. The defined content range is most appropriate to the actions to the skin and most acceptable to make the formation of cataplasma easier. Accordingly, when the content of purified water or glycerin is out of the defined range, it is rather too insignificant to make an exfoliating effect or to form cataplasma with ease. Particularly when the content of the purified water is excessively high, it helps the formation of cataplasma but makes the cataplasma dried out rapidly, shortening the effective time of the cataplasma for the skin. For this reason, it is necessary to use the purified water in an appropriate amount in harmony with glycerin.

The cataplasma components as used herein include a hydrophilic polymer, a polyhydric alcohol, a cross-linking agent, and optionally, other functional additives as needed. In this regard, the individual components and their contents may be appropriately selected as known in the related art.

For example, the hydrophilic polymer as used herein may include at least one selected from the group consisting of gelatin, polyacrylate or its salts, polymethacrylate, carboxyvinyl polymer, polyvinyl alcohol, polyacrylamide, polyethylene oxide, polyethylene imine, polyvinylpyrrolidone, methylcellulose, cellulose, carboxymethylcellulose, and hydroxyethylcellulose.

The polyhydric alcohol as used herein may include at least one selected from the group consisting of glycerin, ethylene glycol, propylene glycol, dipropylene glycol, and butylene glycol.

The cross-linking agent as used herein is preferably a polyvalent metal salt. For example, the cross-linking agent may include at least one selected from the group consisting of aluminum hydroxide, aluminum hydroxide gel, hydrous aluminum silicate, kaolin, aluminum acetate, aluminum lactate, aluminum stearate, calcium chloride, magnesium chloride, aluminum chloride, magnesium aluminometasilicate, and magnesium aluminosilicate.

The above-described components of the present invention have a great technical significance in the three aspects as follows.

Firstly, the components have a good exfoliating action on the skin such as of the heels with the relatively thick cornified layer, yet cause little irritation to the skin. Secondly, the components can be used in combination with a formula called "cataplasma composition", only to further promote the exfoliating effect and maintain the promoted exfoliating effect. Finally, it is possible to find out a novel composition of the components that are applicable as a coating without specific problems on a waterproof fabric sheet, which is used an auxiliary means to help a sufficiently large quantity of exfoliating components effectively penetrate into the target skin area and stay in the topical skin area for a long time.

### [Patch]

The present invention is also directed to a patch having a coating of the above-described cataplasma composition according to the present invention as applied on the one side of a support. In this regard, the support is preferably a waterproof fabric sheet in order to minimize the exfoliating effect.

The reason is that the use of a normal fabric sheet may cause the cataplasma composition to leak out of the support, as shown in the left side of FIG. 1, in the case of an insufficient capture of the oil. When the oil is sufficiently captured in order to avoid this problem, it gets stuck in the gel and fails to make a satisfactory exfoliating effect.

Unlikely, the present invention uses a waterproof fabric sheet as the support to completely prevent the leaks of the oil, as shown in the right side of FIG. 1. Further, the use of the waterproof fabric sheet can prevent the evaporation of water contained in the hydrogel and help the water penetrate into the skin, thus expecting an increase in the exfoliating and moisturizing effects. In a practical experiment, the patch of the present invention with a coating of the cataplasma composition on a waterproof fabric sheet actually has more water penetrate into the skin by at least 20 % than the patch using a normal fabric sheet in 8 hours of the topical application, as shown in Table 1.

**[Table 1]**

| | Waterproof fabric sheet | | Normal fabric sheet | |
|---|---|---|---|---|
| | Weight change | Water loss | Weight change | Water loss |
| 0 min | 100.00 % | | 100.00 % | |
| 60 min | 58.47 % | 41.53 % | 45.61 % | 54.39 % |
| 120 min | 56.27 % | 43.73 % | 44.78 % | 55.22 % |
| 180 min | 55.66 % | 44.34 % | 44.47 % | 55.53 % |
| 480 min | 53.11 % | 46.89 % | 32.95 % | 67.05 % |

If not specifically limited, the waterproof fabric sheet may be prepared by adhering a PE or PU film to a fabric sheet made of PE.

In the case that the patch of the present invention is prepared for exfoliation on the elbow, the support is preferably designed in the shape of FIG. 1 in view of the comfortable application of the patch.

The heel is formed in the shape of " 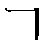 " with a concave curve on either side. For this reason, there are needed two rectangular patches in total, the one on the sole and the other on the back of the foot, in order to cover all the " 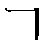 "-shaped heel, as illustrated in the top left side of FIG. 2. In this case, the two patches inevitably overlap each other and thus cannot be tightly and evenly adhered to the target skin area. Further, the rectangular patches are incompetent to cover the concaved heel, as shown in the top left side of FIG. 2.

In contrast, the novel patch design of the present invention can cover the whole surface of the heel evenly and tightly, as shown in the top right side of FIG. 2. Accordingly, this design helps the exfoliating components penetrate into the skin stably and effectively and stay for a long time and allows the patch to tightly adhere to the heel rather than become loose and come off the heel due to active motions.

### [Kit]

The present invention is also directed to a kit comprising the above-described patch of the present invention and an elastic net fixer. The patch of the present invention needs to stick to the heel for a defined period of time or longer in order to maximize its exfoliating effect. In this regard, it is preferable to use a net fixer as an auxiliary means for fixing the patch to the topical application area.

The net fixer can be used without limitation in its type and design as long as it is elastic enough to tightly cover the curvature of the heel and fix the patch on the heel, as shown in the bottom side of FIG. 2.

Hereinafter, the present invention will be described in further detail with reference to the following example, which is given only to explain a preferred embodiment of the present invention and not construed to limit the scope of the present invention.

### <Example>

The following procedures are performed to prepare a patch for softening dead skin cells using the composition of Table 2.
(1) Heat up an aqueous solution of auxiliary polymer PVA to dissolve the polymer and then cool it down. Add a dye prior to the final agitation.
(2) Prepare a catalyst solution.
(3) Prepare a solution of active ingredients (urea and lactic acid).
(4) Homogeneously disperse all polymers and insoluble ingredients in glycerin to prepare a glycerin paste.
(5) Add all natural moisturizing oils and vitamins to 1,3-butylene glycol to prepare a natural moisturizing oil solution.
(6) Homogeneously mix the oil layers of the steps (4) and (5) in a main mixer.
(7) Add the auxiliary polymer solution to the mixture of the step (6) under the condition that the mixing temperature is controlled not to exceed 30 °C.
(8) Adds the aqueous solutions of the steps (3) and (2) to the mixture of the step (7), under the condition that the mixing temperature is raised but maintained at 25 to 30 °C.
(9) After the completion of the homogeneous mixture, perform the reverse roll coating method to apply the active solution evenly on a release film and combine the release film with a waterproof fabric sheet.
(10) Cut out the laminated cataplasma.
(11) Keep the cataplasma in an aging chamber for one to two days.
(12) Package the aged cataplasma.

**[Table 2]**

| Composition (%) | Example | Comparative Example |
|---|---|---|
| 1,3-butylene glycol | 5.00 | 5.00 |
| Glycerin | 13.00 | 13.00 |
| Olive oil | 3.00 | 3.00 |
| Jojoba oil | 3.00 | 3.00 |
| Lactic acid | 1.00 | - |
| Fumaric acid | - | 1.00 |
| Urea | 9.50 | 9.50 |
| Methyl paraben | 0.10 | 010 |
| Propyl paraben | 0.05 | 0.05 |
| EDTA-2Na | 0.10 | 010 |
| CMC-Na | 3.00 | 3.00 |
| Vitamin E | 0.50 | 0.50 |
| Twin 80 | 0.50 | 0.50 |
| Aluminum glycinate | 0.18 | 0.18 |
| Partially neutralized polyacrylate | 4.00 | 4.00 |
| Polyacrylate | 2.00 | 2.00 |
| Flavor oil | 0.40 | 0.40 |
| Polyvinyl alcohol | 3.00 | 3.00 |
| Tartaric acid | 0.40 | 0.40 |
| Colorant | 0.06 | 0.06 |
| Purified water | 51.21 | 51.21 |

A comparison of the effect between Example and Comparative Example is shown in FIG. 3.

It is revealed that the patch product containing a cataplasma composition using a combination of lactic acid and a natural moisturizing oil is far superior to the patch product containing a cataplasma composition using a combination of fumaric acid and a natural oil in terms of the skin softening effect and the moisturizing effect and the retention power for the effects on the skin such as of the heel with the cornified layer relatively thick and hard.

In addition, twenty volunteers are recruited to topically apply the patch product of the present invention containing the cataplasma composition or the conventional patch product not containing the cataplasma composition alternately every one week for 4 weeks and evaluate the application feeling (irritation, moistness and softness) and the feeling of use (immobilization or the like). According to the experimental results, the patch product of the present invention is more highly evaluated than the conventional patch product.

On the other hand, the composition of KR Laid-Open Patent Publication No. 10-2011-0013000 makes no difference before and after the use in terms of the moisture content of the skin as evaluated in one to two hours after the topical application of the active solution. Contrarily, the composition of the present invention shows the moisture content of the skin increased by about 10 to 20 % in one to two hours after its topical application. Besides, the use of potassium lactate according to KR Laid-Open Patent Publication No. 10-2011-0013000 results in a serious problem in the formation of cataplasma. More specifically, the cross-linking reaction is so fast as to make the preparation of cataplasma difficult. In the conception first contrived by the inventors of the present invention, the intended effects of the present invention are impossible to achieve satisfactorily without the formation of cataplasma. This explicitly shows the limits of the conventional composition according to KR Laid-Open Patent Publication No. 10-2011-0013000.

## Claims

1. A patch for exfoliation comprising a coating of a cataplasma composition for exfoliation applied on the one side of a support, said cataplasma composition comprising 0.5 to 1.0 wt.% of lactic acid, natural moisturizing oil, 5 to 10 wt.% of urea, a hydrophilic polymer, a polyhydric alcohol, and a cross-linking agent wherein the natural moisturizing oil includes at least one selected from the group consisting of olive oil, jojoba oil derived from jojoba seeds, and tea tree oil derived from tea tree leaves and wherein said cross-linking agent is a polyvalent metal salt.

2. The patch for exfoliation as claimed in claim 1, wherein the support is a waterproof fiber sheet.

3. The patch as claimed in any one of claims 1 or 2, wherein said polyhydric alcohol is at least one selected from the group consisting of glycerin, ethylene glycol, propylene glycol, dipropylene glycol, and butylene glycol.

4. The patch as claimed in any one of claims 1 to 3, comprising 40 to 50 wt.% of purified water and 10 to 20 wt.% of glycerin with respect to the total weight of the composition.

5. The patch as claimed in any one of claims 1 to 4, wherein the composition comprises 1 to 5 wt.% of olive oil, 1 to 5 wt.% of jojoba oil derived from jojoba seeds, and 0.1 to 0.5 wt.% of tea tree oil derived from tea tree leaves with respect to the total weight of the composition.

6. The patch as claimed in any one of claims 1 to 5, wherein the hydrophilic polymer includes at least one selected from the group consisting of gelatin, polyacrylate or its salts, polymethacrylate, carboxyvinyl polymer, polyvinyl alcohol, polyacrylamide, polyethylene oxide, polyethylene imine, polyvinylpyrrolidone, methylcellulose, cellulose, carboxymethylcellulose, and hydroxyethylcellulose.

7. The patch as claimed in any one of claims 1 to 6, wherein said polyvalent metal salt includes at least one selected from the group consisting of aluminum hydroxide, aluminum hydroxide gel, hydrous aluminum silicate, kaolin, aluminum acetate, aluminum lactate, aluminum stearate, calcium chloride, magnesium chloride, aluminum chloride, magnesium aluminometasilicate, and magnesium aluminosilicate.

8. A kit for exfoliation comprising the patch as claimed in any one of claims 1 to 7 and an elastic net fixer.

## Patentansprüche

1. Pflaster für Exfoliation, umfassend eine Beschichtung mit einer Kataplasma-Zusammensetzung für Exfoliation, die auf eine Seite eines Trägers aufgebracht ist, wobei die Kataplasma-Zusammensetzung 0,5 bis 1,0 Gew.-% Milchsäure, natürliches feuchtigkeitsspendendes Öl, 5 bis 10 Gew.-% Harnstoff, ein hydrophiles Polymer, einen mehrwertigen Alkohol und ein Vernetzungsmittel umfasst, wobei das natürliche feuchtigkeitsspendende Öl mindestens eines ausgewählt aus der aus Olivenöl, aus Jojobasamen stammendes Jojobaöl und aus Teebaumblättern stammendes Teebaumöl bestehenden Gruppe umfasst, und wobei es sich beim Vernetzungsmittel um ein mehrwertiges Metallsalz handelt.

2. Pflaster für Exfoliation nach Anspruch 1, wobei es sich beim Träger um einen wasserfesten Faserstoff handelt.

3. Pflaster nach einem der Ansprüche 1 oder 2, wobei der mehrwertige Alkohol mindestens einer ausgewählt aus der aus Glycerin, Ethylenglykol, Propylenglykol, Dipropylenglykol und Butylenglykol bestehenden Gruppe ist.

4. Pflaster nach einem der Ansprüche 1 bis 3, umfassend 40 bis 50 Gew.-% gereinigtes Wasser und 10 bis 20 Gew.-% Glycerin, bezogen auf das Gesamtgewicht der Zusammensetzung.

5. Pflaster nach einem der Ansprüche 1 bis 4, wobei die Zusammensetzung 1 bis 5 Gew.-% Olivenöl, 1 bis 5 Gew.-% aus Jojobasamen stammendes Jojobaöl, 0,1 bis 0,5 Gew.-% aus Teebaumblättern stammendes Teebaumöl, bezogen auf das Gesamtgewicht der Zusammensetzung, umfasst.

6. Pflaster nach einem der Ansprüche 1 bis 5, wobei das hydrophile Polymer mindestens eines ausgewählt aus der aus Gelatine, Polyacrylat oder dessen Salzen, Polymethacrylat, Carboxyvinyl-Polymer, Polyvinylalkohol, Polyacrylamid, Polyethylenoxid, Polyethylenimin, Polyvinylpyrrolidon, Methylcellulose, Cellulose, Carboxymethylcellulose und Hydroxyethylcellulose bestehenden Gruppe umfasst.

7. Pflaster nach einem der Ansprüche 1 bis 6, wobei das mehrwertige Metallsalz mindestens eines ausgewählt aus der aus Aluminiumhydroxid, Aluminiumhydroxid-Gel, wasserhaltigem Aluminiumsilicat, Kaolin, Aluminiumacetat, Aluminiumlactat, Aluminiumstearat, Calciumchlorid, Magnesiumchlorid, Aluminiumchlorid, Magnesiumaluminometasilicat und Magnesiumaluminosilicat bestehenden Gruppe umfasst.

8. Kit für Exfoliation, umfassend das Pflaster nach einem der Ansprüche 1 bis 7 und ein elastisches Netzfixiermittel.

## Revendications

1. Un timbre d'exfoliation comprenant un revêtement en une composition de cataplasme pour exfoliation appliqué sur un premier côté d'un support, ladite composition de cataplasme comprenant 0,5 à 1,0 % en poids d'acide lactique, une huile hydratante naturelle, 5 à 10 % en poids d'urée, un polymère hydrophile, un alcool polyvalent, et un agent de réticulation, dans lequel l'huile hydratante naturelle comprend au moins l'une choisie dans le groupe constitué par l'huile d'olive, l'huile de jojoba dérivée de graines de jojoba, et l'huile d'arbre à thé dérivée de feuilles d'arbre à thé, et dans lequel ledit agent de réticulation est un sel d'un métal polyvalent.

2. Le timbre d'exfoliation tel que revendiqué dans la revendication 1, dans lequel le support est une feuille fibreuse résistante à l'eau.

3. Le timbre tel que revendiqué dans l'une quelconque des revendications 1 et 2, dans lequel ledit alcool polyvalent est au moins l'un choisi dans le groupe constitué par le glycérol, l'éthylèneglycol, le propylèneglycol, le dipropylèneglycol, et le butylèneglycol.

4. Le timbre tel que revendiqué dans l'une quelconque des revendications 1 à 3, comprenant 40 à 50 % en poids d'eau purifiée et 10 à 20 % en poids de glycérol par rapport au poids total de la composition.

5. Le timbre tel que revendiqué dans l'une quelconque des revendications 1 à 4, dans lequel la composition comprend 1 à 5 % en poids d'huile d'olive, 1 à 5 % en poids d'huile de jojoba dérivée de graines de jojoba, et 0,1 à 0,5 % en poids d'huile d'arbre à thé dérivée de feuilles d'arbre à thé par rapport au poids total de la composition.

6. Le timbre tel que revendiqué dans l'une quelconque des revendications 1 à 5, dans lequel le polymère hydrophile comprend au moins l'un choisi dans le groupe constitué par la gélatine, un polyacrylate ou ses sels, un polyméthacrylate, un polymère carboxyvinylique, un poly(alcool vinylique), un polyacrylamide, un poly(oxyde d'éthylène), une polyéthylène-imine, une polyvinylpyrrolidone, la méthylcellulose, la cellulose, la carboxyméthylcellulose, et l'hydroxyéthylcellulose.

7. Le timbre tel que revendiqué dans l'une quelconque des revendications 1 à 6, dans lequel ledit sel d'un métal polyvalent comprend au moins l'un choisi dans le groupe constitué par l'hydroxyde d'aluminium, un gel d'hydroxyde d'aluminium, le silicate d'aluminium hydraté, le kaolin, l'acétate d'aluminium, le lactate d'aluminium, le stéarate d'aluminium, le chlorure de calcium, le chlorure de magnésium, le chlorure d'aluminium, l'aluminométasilicate de magnésium, et l'aluminosilicate de magnésium.

8. Trousse d'exfoliation comprenant le timbre tel que revendiqué dans l'une quelconque des revendications 1 à 7 et un fixateur en filet élastique.
